# EUROPEAN PATENT SPECIFICATION

(11) **EP 3 968 862 B1**
(45) Date of publication and mention of the grant of the patent: **12.07.2023**
(21) Application number: 20726092.8
(22) Date of filing: 14.05.2020
(51) Int. Cl.: A61B 5/00, A61B 8/12, A61B 8/08, A61B 8/00, A61B 5/107

(54) **SYSTEM, DEVICE AND METHOD FOR ASSISTANCE WITH CERVICAL ULTRASOUND EXAMINATION**
SYSTEM, VORRICHTUNG UND VERFAHREN ZUR UNTERSTÜTZUNG VON ULTRASCHALLUNTERSUCHUNG DES GEBÄRMUTTERHALSES
SYSTÈME, DISPOSITIF ET PROCÉDÉ D'ASSISTANCE À UN EXAMEN DU COL DE L'UTÉRUS PAR ULTRASONS

(30) Priority: 17.05.2019 US 201962849219 P
(43) Date of publication of application: 23.03.2022
(73) Proprietor: Koninklijke Philips N.V., 5656 AG Eindhoven (NL)
(72) Inventor: SRINIVASA NAIDU, Raghavendra, 5656 AE Eindhoven (NL); BHARAT, Shyam, 5656 AE Eindhoven (NL); ERRICO, Claudia, 5656 AE Eindhoven (NL)
(74) Representative: Philips Intellectual Property & Standards
(86) International application number: PCT/EP2020/063448
(87) International publication number: WO 2020/234106

(56) References cited:
- US-A1- 2016 166 233
- US-A1- 2018 103 912
- US-A1- 2018 199 919

## Description

### TECHNICAL FIELD

This invention pertains to acoustic (e.g., ultrasound) imaging, and in particular a system, device and method for assistance with a cervical ultrasound examination.

### BACKGROUND AND SUMMARY

US 2016/166233 A1 discloses an ultrasound diagnosis apparatus that measures a pressure value applied from a probe to a cervix to more accurately measure a cervix length.

Acoustic (e.g., ultrasound) imaging systems are increasingly being employed in a variety of applications and contexts. For example, acoustic imaging is increasingly being employed in the context of cervical examination.

Cervical-length measurement using transvaginal sonography (TVS) is an essential part of assessing the risk of preterm delivery. At mid-gestation, it provides a useful method with which to predict the likelihood of subsequent preterm birth in asymptotic women. There are essentially four methods that have been used to evaluate the uterine cervix: digital examination, transabdominal ultrasound, transperineal ultrasound, and transvaginal sonography (TVS).

Digital examinations suffer from being subjective, and have low accuracy in measuring the cervical length. Acoustic (e.g., ultrasound) imaging makes an ideal modality with which to address both of these challenges due to its ability to visualize cervical tissue in a minimally invasive manner.

However, obtaining the right view of the cervix, having accurate measurements (caliper placement), and correct identification of anatomical landmarks remain very challenging.

Accordingly, it would be desirable to provide a system and a method which can address these challenges in cervical ultrasound imaging. It would also be desirable to provide guidance to sonographers to identify the right imaging plane, and the cervix funnel anatomical landmark, and to perform accurate measurements of cervical length during pregnancy.

In one aspect of the invention, a system comprises an acoustic probe having an array of acoustic transducer elements; an inertial measurement unit configured to provide an inertial measurement signal indicating a pose of the acoustic probe; and an acoustic imaging instrument connected to the acoustic probe and configured to provide transmit signals to least some of the acoustic transducer elements to cause the array of acoustic transducer elements to transmit an acoustic probe signal to an area of interest including a cervix, and further configured to produce acoustic images of the area of interest in response to acoustic echoes received by the acoustic probe from the area of interest in response to the acoustic probe signal. The acoustic imaging instrument includes: a display device; a communication interface configured to receive one or more image signals from the acoustic probe produced from the acoustic echoes from the area of interest, and to receive the inertial measurement signal; and a processor, and associated memory. The processor is configured to, for each of a plurality of time frames in a scan session: construct a three dimensional volume of the area of interest from the one or more image signals and the received inertial measurement signal, apply a deep learning algorithm to the constructed three dimensional volume of the area of interest to qualify an image plane for obtaining a candidate cervical length for the cervix, and perform image segmentation and object detection for the qualified image plane to obtain the candidate cervical length. The processor is configured to select the shortest candidate cervical length from the plurality of time frames as a measured cervical length for the scan session, and to control the display device to display an image of the cervix corresponding to the measured cervical length for the scan session, and an indication of the measured cervical length for the scan session.

In some embodiments, the processor is configured to control the display device to display a graph showing the candidate cervical lengths and to display the indication of the measured cervical length for the scan session on the graph.

In some embodiments, the processor is configured to store in a nonvolatile memory device the measured cervical length for the scan session and a date of the scan session.

In some embodiments, the nonvolatile memory device is configured to store in the nonvolatile memory device a plurality of measured cervical lengths for a plurality of scan sessions performed at corresponding times, and wherein the processor is configured to cause the display to display a graph plotting the cervical lengths for the scan sessions against the corresponding times.

In some embodiments, the processor is configured to generate image data for the qualified image plane and to perform image segmentation by applying the image data for the qualified image plane to a You Only Look Once (YOLO) neural network.

In some embodiments, the processor is configured to generate image data for the qualified image plane and to perform object detection for the qualified image plane by applying the image data for the qualified image plane to a U-Net Convolutional network.

In some embodiments, the processor is configured to generate image data for a plurality of image planes of the three dimensional volume, and wherein the deep learning algorithm employs one or more qualifying anatomical landmarks which qualify image planes of the three dimensional volume, and employs one or more disqualifying anatomical landmarks which disqualify image planes of the three dimensional volume.

In some embodiments, a first cervical shape is employed as one of the disqualifying anatomical landmarks and a second cervical shape is employed as one of the qualifying anatomical landmarks. In particular, certain anatomical landmarks, such as certain cervical shapes, indicate that the view is not a good view for measuring cervical length, in which case that view is disqualified for being used for cervical length measurements.

In some embodiments, the processor is configured to generate image data for a plurality of image planes of the three dimensional volume, and wherein the deep learning algorithm applies the image data to one of a convolutional neural network (CNN), a You Only Look Once (YOLO) neural network, or a U-Net Convolutional network.

In another aspect of the invention, a method includes performing real time two-dimensional acoustic imaging of an area of interest during a scan session, including a cervix, with an acoustic probe, including producing one or more image signals of the area of interest and producing an inertial measurement signal indicating a pose of the acoustic probe. The method further includes, for each of a plurality of time frames in the scan session: constructing a three dimensional volume of the area of interest from the one or more image signals and the inertial measurement signal, applying a deep learning algorithm to the constructed three dimensional volume of the area of interest to qualify an image plane for obtaining a candidate cervical length for the cervix, and performing image segmentation and object detection for the qualified image plane to obtain the candidate cervical length. The method further includes selecting a shortest candidate cervical length from the plurality of time frames as a measured cervical length for the scan session, and displaying on a display device an image of the cervix corresponding to the measured cervical length for the scan session, and an indication of the measured cervical length for the scan session.

In some embodiments, the method further comprises displaying a graph showing the candidate cervical lengths and displaying the indication of the measured cervical length for the scan session on the graph.

In some embodiments, the method further comprises storing the measured cervical length for the scan session and a date of the scan session in a nonvolatile memory device.

In some embodiments, the method further comprises: storing in the nonvolatile memory device a plurality of measured cervical lengths for a plurality of scan sessions performed at corresponding times; and displaying on the display device a graph plotting the cervical lengths for the scan sessions against the corresponding times.

In some embodiments, the method further comprises generating image data for the qualified image plane and performing image segmentation by applying the image data for the qualified image plane to a You Only Look Once (YOLO) neural network.

In some embodiments, the method further comprises: generating image data for the qualified image plane; and performing object detection for the qualified image plane by applying the image data for the qualified image plane to a U-Net Convolutional network.

In some embodiments, the method further comprises: generating image data for a plurality of image planes of the three dimensional volume; employing one or more qualifying anatomical landmarks which qualify image planes of the three dimensional volume; and employing one or more disqualifying anatomical landmarks which disqualify image planes of the three dimensional volume in order.

In some embodiments, the method further comprises: employing a first cervical shape as one of the disqualifying anatomical landmarks; and employing a second cervical shape as one of the qualifying anatomical landmarks.

In some embodiments, the method further comprises: generating image data for a plurality of image planes of the three dimensional volume; and applying the image data to one of a convolutional neural network (CNN), a You Only Look Once (YOLO) neural network, or a U-Net Convolutional network to qualify an image plane for obtaining a candidate cervical length for the cervix.

### BRIEF DESCRIPTION OF THE DRAWINGS

FIG. 1 illustrates possible clinical pathways for pregnancy based on cervical length assessment.
FIG. 2A shows an acoustic image of a desired view of a cervix with anatomical landmarks.
FIG. 2B illustrates a pictorial view of a typical anatomy of the cervix.
FIG. 3 illustrates example acoustic images of different funneling patterns for a cervix.
FIG. 4 illustrates an example of an acoustic image with a suboptimal view of a cervix for determining cervical length.
FIG. 5 illustrates an example of an acoustic image of a cervix with inaccurate cursor placement for determining cervical length.
FIG. 6 illustrates an example of an acoustic image of a cervix produced with excess pressure by the acoustic probe on a cervix.
FIG. 7 illustrates an example of an acoustic image of a cervix depicting contractions.
FIG. 8 illustrates an example embodiment of an acoustic imaging apparatus.
FIG. 9 is a block diagram illustrating an example processing unit according to embodiments of the disclosure.
FIG. 10 illustrates an example embodiment of an acoustic probe.
FIG. 11 illustrates an example operation of an acoustic imaging apparatus.
FIGs. 12A, 12B and 12C illustrate an example operation of a process of constructing a three dimensional (3D) volume from a series of two-dimensional acoustic images.
FIG. 13 illustrates major operations in an example embodiment of an algorithm for determining the cervical length of a cervix.
FIG. 14A illustrates a graph which may be displayed in a user interface to show candidate cervical lengths and to indicate the measured cervical length for a scan session.
FIG. 14B illustrates a graph which may be displayed in a user interface to show a progression of measured cervical lengths over time from multiple scan sessions during a pregnancy.
FIG. 15 illustrates a flowchart of an example embodiment of a method of determining the cervical length of a cervix.

### DETAILED DESCRIPTION

The present invention will now be described more fully hereinafter with reference to the accompanying drawings, in which preferred embodiments of the invention are shown. This invention may, however, be embodied in different forms and should not be construed as limited to the embodiments set forth herein. Rather, these embodiments are provided as teaching examples of the invention. Herein, when something is said to be "approximately" or "about" a certain value, it means within 10% of that value.

Preterm birth (PTB) remains a major cause of perinatal morbidity and mortality, and so its prediction and prevention are two of the most important issues in obstetrics. Cervical weakness (incompetence) is a medical condition that causes preterm birth.

To diagnose this condition, cervical length (CL) may be measured using an acoustic (e.g., ultrasound) imaging system. Acoustic imaging has been shown to be the best predictor of preterm birth. At mid-gestation, acoustic imaging provides a useful method with which to predict the likelihood of subsequent preterm birth in asymptomatic women. In women who present with symptoms of spontaneous preterm labor, measurement of cervical length can help to distinguish between 'true' and 'false' spontaneous (cervix opens prematurely with no contractions ) preterm labor. Additionally, there is some evidence that measurement of the cervix at the 11±0 and 13± week scan can help establish the risk of preterm birth.

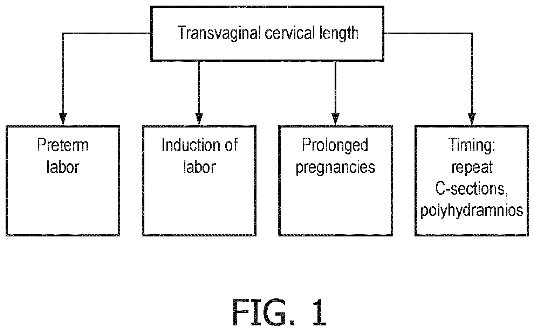

FIG. 1 illustrates possible clinical pathways for pregnancy based on cervical length assessment. In particular, FIG. 1 illustrates a number of problems in pregnancy which have been associated with suboptimal cervical lengths, including preterm labor, the need to induce labor, prolonged pregnancies, and the need for repeated C-sections. These problems are not associated with normal pregnancy outcomes. For example, one study reports that when the cervical length is less than 2.2 cm, women face a 20 percent probability of preterm delivery. Also, increased cervical length late in pregnancy has been correlated to prolonged pregnancies.

The American College of Obstetricians and Gynecologists (ACOG) and the Society for Maternal-Fetal Medicine (SMFM) recommend that cervical length (CL) be measured every 2 weeks during pregnancy from 16 to 23 weeks in singletons with prior spontaneous PTB (sPTB), with cerclage placed for CL less than 25 mm.

As noted above, there are essentially four methods that can be used to evaluate the uterine cervix: digital examination, transabdominal ultrasound, transperineal ultrasound and transvaginal ultrasound (TVS). The digital examination provides the most comprehensive evaluation of the cervix, assessing dilation, position, consistency and length. However, this examination suffers from being subjective. It is limited especially in its ability to accurately establish the cervical length. It also cannot reproducibly detect any changes at the internal cervical os and upper portion of the cervical canal. Acoustic (e.g., ultrasound) imaging, with its ability to visualize the cervical tissue and display its anatomy, makes an ideal modality with which to address both of these issues.

To ensure correct measurements, a transvaginal probe is inserted for a first assessment of the anatomy of the cervix, then it is withdrawn until the acoustic image blurs (makes dim or dark images) to reduce compression from the transducer. Eventually it is moved forward again to reapply just enough pressure to create the best image. Obtaining the right image view and procedure requires applying mild suprapubic or fundal pressure for approximately 15 seconds to watch for funneling (shortening of the top portion of the cervix). The probe pressure is then reduced while fundal or suprapubic pressure is applied. Then three measurements are obtained and the shortest one is usually recorded.

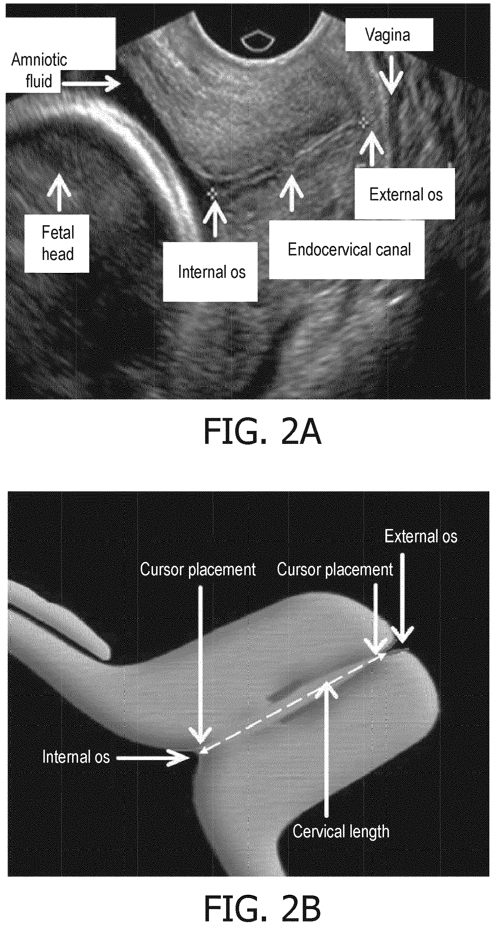

FIG. 2A shows an acoustic image of a desired view of a cervix with anatomical landmarks, and FIG. 2B illustrates a pictorial view of a typical anatomy of the cervix. The typical anatomy shows an internal and an external os. The cervical length is measured between these two points.

In addition to cervical length measurement, sonographers have to look for additional significant findings such as funneling, defined as protrusion of the amniotic membranes into the cervical canal. Cervical funneling is a sign of cervical incompetence and represents the dilatation of the internal part of the cervical canal and reduction of the cervical length. The specific funneling pattern indicates the risk of preterm birth. Greater than 50% funneling before 25 weeks is associated with approximately 80% risk of preterm delivery (https://radiopaedia.org/articles/funneling-of-the-internal-cervical-os).

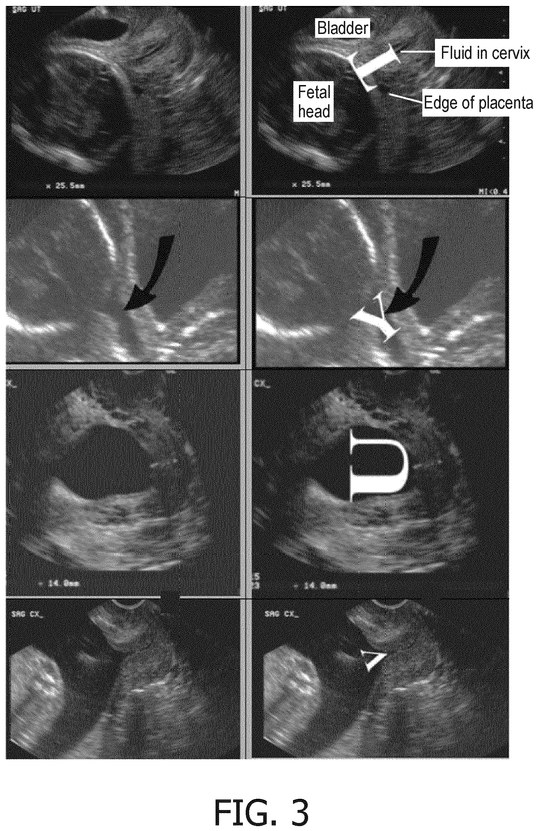

FIG. 3 illustrates example acoustic images of different funneling patterns for a cervix. Different funneling patterns may occur due to the skill of the operator and the position of the fetus. One significant factor is the amount of pressure applied to the cervix by the operator. Likewise the estimated cervix length can change due to a number of reasons, including patient motion, breathing, probe motion etc.

Though ultrasound imaging is the best modality of choice for the measurement of cervical length, ultrasonography remains an operator-dependent modality, and many pitfalls are possible with regard to image technique or interpretation. The radiologist should be able to recognize these imaging findings related to the risk of preterm birth and report them to the referring clinician. The clinician may then select patients who should undergo serial ultrasound studies from the start of the second trimester of pregnancy, or determine suitable treatment based on the ultrasound findings suggestive of incompetence before clinical examination.

In order for the cervical length measurement to be accurate and reproducible, several factors need to be taken into account. In particular, repeated TVS measurements of the cervix need to be made and they should meet several criteria, for example each cervical length measurement should differ by less than 10%. Of the best cervical length measurements, sonographers should record the shortest cervical length measurement.

Some common sources of error, which can lead to inaccurate measurements, are described below.

First, it is important to be able to visualize the entire cervix from the acoustic image(s).

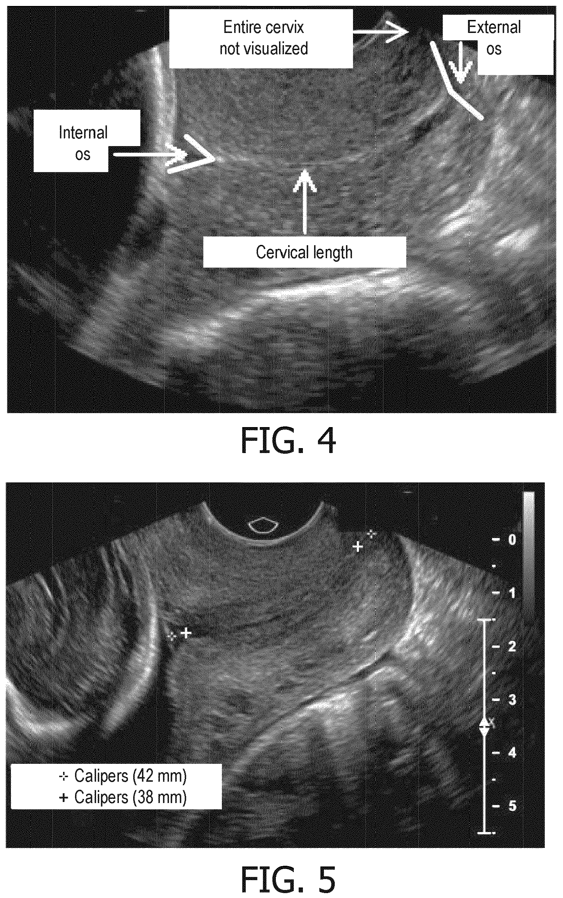

FIG. 4 illustrates an example of an acoustic image with a suboptimal view of a cervix for determining cervical length. In the example image of FIG. 4, the entire cervix is not visualized, and the internal and external os is not well defined. Although the cervical length is probably normal, this is a suboptimal image.

It is also important to accurately place the measurement calipers in the image.

FIG. 5 illustrates an example of an acoustic image of a cervix where the placement of the caliper is not exact and the distal cervix is not completely visualized, which hampers the recognition of the external cervical os.

Additionally, it is important to produce the acoustic image(s) of the cervix without causing the acoustic probe to apply excess pressure to the cervix.

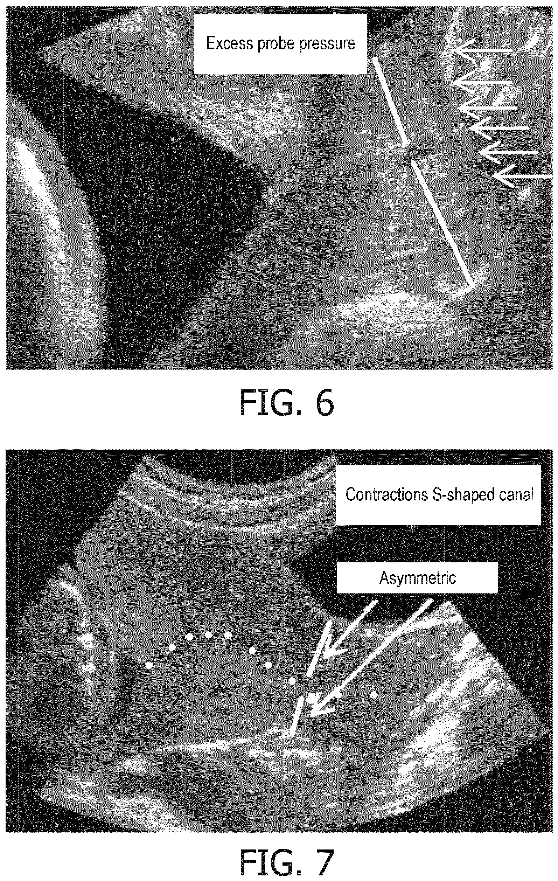

FIG. 6 illustrates an example of an acoustic image of a cervix produced with excess pressure by the acoustic probe on a cervix. In particular, FIG. 6 shows dissimilarities between the thickness of the anterior and posterior cervical lips due to excess pressure by the acoustic prove on the cervix during imaging.

FIG. 7 illustrates an example of an acoustic image of a cervix depicting contractions. FIG. 7 shows how contractions lead to an s-shaped canal and asymmetry of the anterior portions of the cervix.

To address one or more of these problems, an artificial intelligence (AI)/deep learning based system is employed in systems and methods described below, to enable an accurate cervical measurement. In some embodiments, these systems and methods may:
- Identify anatomical landmarks and provide visual feedback to the user.
- Based on the anatomical landmarks, guide the user to maneuver an acoustic probe to obtain an optimal view.
- Identify excessive acoustic probe pressure based on the current acoustic image and provide visual feedback to the user.
- Provide visual feedback to a user about which criteria for obtaining an accurate cervical length measurement have been satisfied (and which ones haven't been satisfied) based on above identified information.
- Guide the user to perform appropriate actions based on the criteria which haven't been satisfied. For example, if a proper view for an accurate cervical length measurement is not identified, the user may be instructed or advised how to maneuver the acoustic probe to achieve the best imaging plane which meets all the criteria for an accurate cervical length measurement.
- Automatically identify the caliper points and record the shortest best cervical length measurement.
- (If the sonographer wants to re-obtain a previously identified scan plane, either within the same scanning session or in a follow-up scanning session) identify the prior scan plane for subsequent measurement using the 3D volume.
- If the current session is the follow up scan, provide a longitudinal summary about the progression of the cervical length over time.

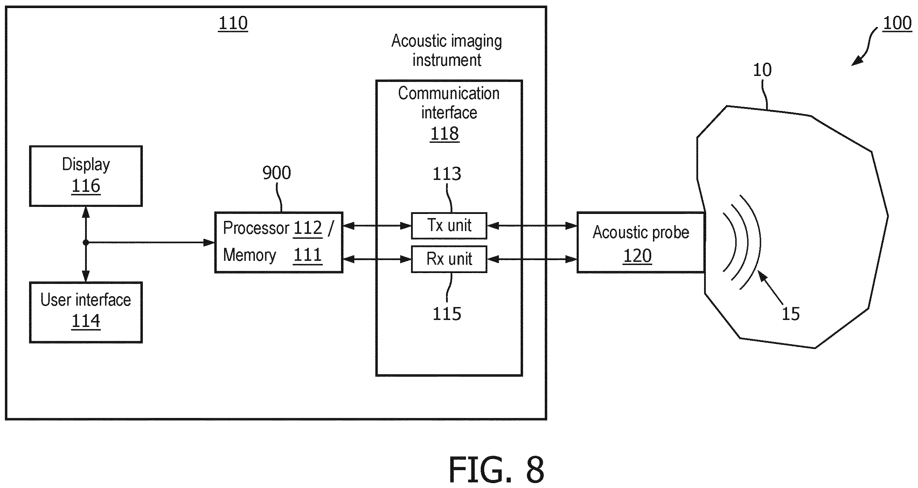

FIG. 8 shows one example of an acoustic imaging system 100 which includes an acoustic imaging instrument 110 and an acoustic probe 120. Acoustic imaging instrument 110 includes a processing unit 900, a user interface 114, a display device 116 and a communication interface 118. Processing unit 900 may include a processor 112 and a memory 111.

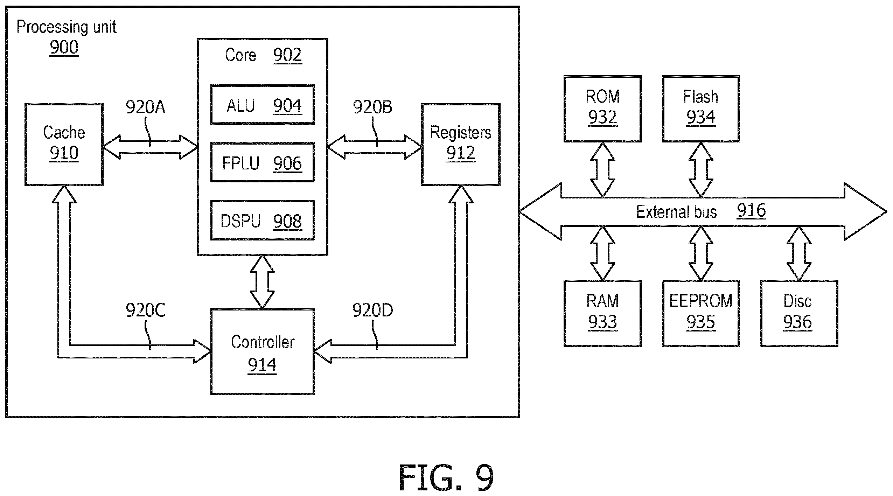

FIG. 9 is a block diagram illustrating an example processing unit 900 according to embodiments of the disclosure. Processing unit 900 may be used to implement one or more processors described herein, for example, processor 112 shown in FIG. 8. Processing unit 900 may be any suitable processor type including, but not limited to, a microprocessor, a microcontroller, a digital signal processor (DSP), a field programmable array (FPGA) where the FPGA has been programmed to form a processor, a graphical processing unit (GPU), an application specific circuit (ASIC) where the ASIC has been designed to form a processor, or a combination thereof.

Processing unit 900 may include one or more cores 902. Core 902 may include one or more arithmetic logic units (ALU) 904. In some embodiments, core 902 may include a floating point logic unit (FPLU) 906 and/or a digital signal processing unit (DSPU) 908 in addition to or instead of the ALU 904.

Processing unit 900 may include one or more registers 912 communicatively coupled to core 902. Registers 912 may be implemented using dedicated logic gate circuits (e.g., flip-flops) and/or any memory technology. In some embodiments the registers 912 may be implemented using static memory. The register may provide data, instructions and addresses to core 902.

In some embodiments, processing unit 900 may include one or more levels of cache memory 910 communicatively coupled to core 902. Cache memory 910 may provide computer-readable instructions to core 902 for execution. Cache memory 910 may provide data for processing by core 902. In some embodiments, the computer-readable instructions may have been provided to cache memory 910 by a local memory, for example, local memory attached to external bus 916. Cache memory 910 may be implemented with any suitable cache memory type, for example, metal-oxide semiconductor (MOS) memory such as static random access memory (SRAM), dynamic random access memory (DRAM), and/or any other suitable memory technology.

Processing unit 900 may include a controller 914, which may control input to the processor 900 from other processors and/or components included in a system (e.g., acoustic imaging system 100 in FIG. 8) and/or outputs from processing unit 900 to other processors and/or components included in the system (e.g., communication interface 118 shown in FIG. 8). Controller 914 may control the data paths in the ALU 904, FPLU 906 and/or DSPU 908. Controller 914 may be implemented as one or more state machines, data paths and/or dedicated control logic. The gates of controller 914 may be implemented as standalone gates, FPGA, ASIC or any other suitable technology.

Registers 912 and the cache 910 may communicate with controller 914 and core 902 via internal connections 920A, 920B, 920C and 920D. Internal connections may be implemented as a bus, multiplexor, crossbar switch, and/or any other suitable connection technology.

Inputs and outputs for processing unit 900 may be provided via a bus 916, which may include one or more conductive lines. The bus 916 may be communicatively coupled to one or more components of processing unit 900, for example the controller 914, cache 910, and/or register 912. The bus 916 may be coupled to one or more components of the system, such as components BBB and CCC mentioned previously.

Bus 916 may be coupled to one or more external memories. The external memories may include Read Only Memory (ROM) 932. ROM 932 may be a masked ROM, Electronically Programmable Read Only Memory (EPROM) or any other suitable technology. The external memory may include Random Access Memory (RAM) 933. RAM 933 may be a static RAM, battery backed up static RAM, Dynamic RAM (DRAM) or any other suitable technology. The external memory may include Electrically Erasable Programmable Read Only Memory (EEPROM) 935. The external memory may include Flash memory 934. The external memory may include a magnetic storage device such as disc 936. In some embodiments, the external memories may be included in a system, such as ultrasound imaging system 100 shown in FIG. 8.

It should be understood that in various embodiments, acoustic imaging system 100 may be configured differently than described below with respect to FIG. 8. In particular, in different embodiments, one or more functions described as being performed by elements of acoustic imaging instrument 110 may instead be performed in acoustic probe 120 depending, for example, on the level of signal processing capabilities which might be present in acoustic probe 120.

In various embodiments, processor 112 may include various combinations of a microprocessor (and associated memory), a digital signal processor, an application specific integrated circuit (ASIC), a field programmable gate array (FPGA), digital circuits and/or analog circuits. Memory (e.g., nonvolatile memory) 111, associated with processor 112, may store therein computer-readable instructions which cause a microprocessor of processor 112 to execute an algorithm to control acoustic imaging system 100 to perform one or more operations or methods which are described in greater detail below. In some embodiments, a microprocessor may execute an operating system. In some embodiments, a microprocessor may execute instructions which present a user of acoustic imaging system 100 with a graphical user interface (GUI) via user interface 114 and display device 116.

In various embodiments, user interface 114 may include any combination of a keyboard, keypad, mouse, trackball, stylus /touch pen, joystick, microphone, speaker, touchscreen, one or more switches, one or more knobs, one or more buttons, one or more lights, etc. In some embodiments, a microprocessor of processor 112 may execute a software algorithm which provides voice recognition of a user's commands via a microphone of user interface 114.

Display device 116 may comprise a display screen of any convenient technology (e.g., liquid crystal display). In some embodiments the display screen may be a touchscreen device, also forming part of user interface 114.

Communication interface 118 includes a transmit unit 113 and a receive unit 115.

Transmit unit 113 may generate one or more electrical transmit signals under control of processor 112 and supply the electrical transmit signals to acoustic probe 120. Transmit unit 113 may include various circuits as are known in the art, such as a clock generator circuit, a delay circuit and a pulse generator circuit, for example. The clock generator circuit may be a circuit for generating a clock signal for setting the transmission timing and the transmission frequency of a drive signal. The delay circuit may be a circuit for setting delay times in transmission timings of drive signals for individual paths corresponding to the transducer elements of acoustic probe 120 and may delay the transmission of the drive signals for the set delay times to concentrate the acoustic beams to produce acoustic probe signal 15 having a desired profile for insonifying a desired image plane. The pulse generator circuit may be a circuit for generating a pulse signal as a drive signal in a predetermined cycle.

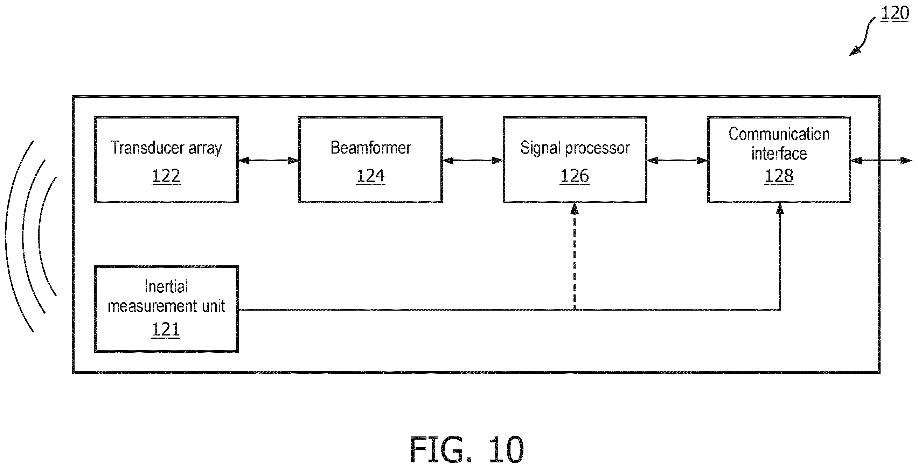

Beneficially, as described below with respect to FIG. 10, acoustic probe 120 may include an array of acoustic transducer elements 122, for example a two dimensional (2D) array or a linear or one dimensional (1D) array. For example, in some embodiments, transducer elements 122 may comprise piezoelectric elements. In operation, at least some of acoustic transducer elements 122 receive electrical transmit signals from transmit unit 113 of acoustic imaging instrument 110 and convert the electrical transmit signals to acoustic beams to cause the array of acoustic transducer elements 122 to transmit an acoustic probe signal 15 to an area of interest 10. Acoustic probe 120 may insonify an image plane in area of interest 10 and a relatively small region on either side of the image plane (i.e., it expands to a shallow field of view).

Also, at least some of acoustic transducer elements 122 of acoustic probe 120 receive acoustic echoes from area of interest 10 in response to acoustic probe signal 15 and convert the received acoustic echoes to one or more electrical signals representing an image of area of interest 10. These electrical signals may be processed further by acoustic probe 120 and communicated by a communication interface of acoustic probe 120 (see FIG. 10) to receive unit 115 as one or more image signals.

Receive unit 115 is configured to receive the one or more image signals from acoustic probe 120 and to process the image signal(s) to produce acoustic image data. In some embodiments, receive unit 115 may include various circuits as are known in the art, such as one or more amplifiers, one or more A/D conversion circuits, and a phasing addition circuit, for example. The amplifiers may be circuits for amplifying the image signals at amplification factors for the individual paths corresponding to the transducer elements 122. The A/D conversion circuits may be circuits for performing analog/digital conversion (A/D conversion) on the amplified image signals. The phasing addition circuit is a circuit for adjusting time phases of the amplified image signals to which A/D conversion is performed by applying the delay times to the individual paths respectively corresponding to the transducer elements 122 and generating acoustic data by adding the adjusted received signals (phase addition). The acoustic data may be stored in memory 111 or another memory associated with acoustic imaging instrument 100.

Processor 112 may reconstruct acoustic data received from receiver unit 115 into an acoustic image corresponding to an image plane which intercepts area of interest 10, and subsequently causes display device 116 to display this image. The reconstructed image may for example be an ultrasound Brightness-mode "B-mode" image, otherwise known as a "2D mode" image, a "C-mode" image or a Doppler mode image, or indeed any ultrasound image.

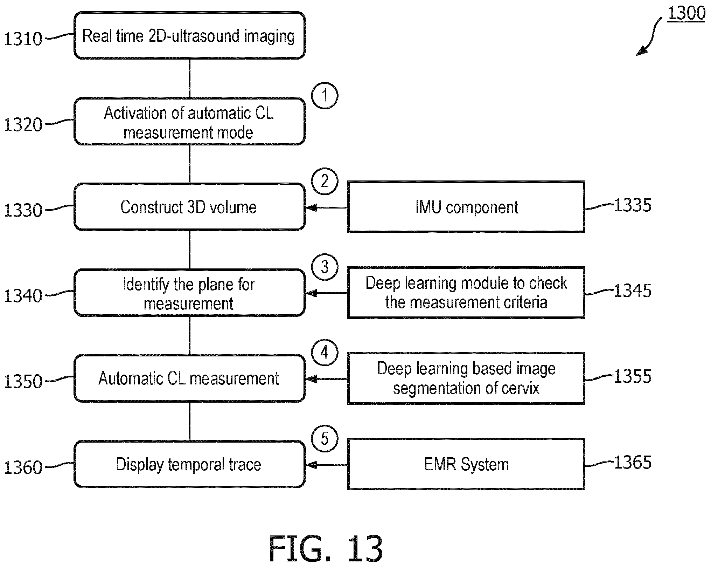

In various embodiments, processor 112 may execute software in one or more modules for performing one or more algorithms or methods as described below with respect to FIGs. 13-15 to measure cervical length in response to image signals received by acoustic probe 120 probing area of interest 10 including a cervix.

Of course it is understood that acoustic imaging instrument 110 may include a number of other elements not shown in FIG. 8, for example a power system for receiving power from AC Mains, an input/output port for communications between processor 112 and acoustic probe 120, a communication subsystem for communicating with other eternal devices and systems (e.g., via a wireless, Ethernet and/or Internet connection), etc.

In some embodiments, acoustic imaging instrument 110 also receives an inertial measurement signal from an inertial measurement unit (IMU) included in or associated with acoustic probe 120. The inertial measurement signal may indicate an orientation or pose of acoustic probe 120. The inertial measurement unit may include a hardware circuit, a hardware sensor or Microelectromechanical systems (MEMS) device. The inertial measurement circuity may include a processing unit, such as processing unit 900, running software in conjunction with a hardware sensor or MEMS device.

FIG. 10 illustrates an example embodiment of acoustic probe 120. In some embodiments, acoustic probe 120 may comprise a transvaginal sonography (TVS) probe for providing an acoustic image of a cervix.

Acoustic probe 120 includes an array of acoustic transducer elements 122, a beamformer 124, a signal processor 126, a communication interface 128, and an inertial measurement unit 121. In some embodiments, inertial measurement unit 121 may be a separate component not included within acoustic probe 120, but associated therewith, such as being affixed to or mounted on acoustic probe 120. Inertial measurement units per se are known. Inertial measurement unit 121 is configured to provide an inertial measurement signal to acoustic imaging instrument 110 which indicates a current orientation or pose of acoustic probe 120 so that a 3D volume may be constructed from a plurality of 2D images obtained with different poses of acoustic probe 120.

Communication interface 128 is connected to signal processor 126 and may also be connected with communication interface 115 of acoustic imaging instrument 110. Signal processor 126 is also connected with beamformer 124. Beamformer 124 is further connected to transducer array 122.

In operation, acoustic imaging instrument 110 may provide to acoustic probe, via communication interface 128, one or more control signals which may be processed as desired by signal processor 126. One or more signals output by signal processor 126 may be supplied to beamformer 124 which in response thereto may supply signals to transducer array to transmit a desired acoustic probe signal 15 to area of interest 10.

Also, at least some of acoustic transducer elements 122 of acoustic probe 120 receive acoustic echoes from area of interest 10 in response to acoustic probe signal 15 and convert the received acoustic echoes to one or more electrical signals representing an image of area of interest 10. These electrical signals may be processed further by beamformer 124 and signal processor 126 as desired and then communicated by communication interface 128 to acoustic imaging instrument 110 as one or more image signals.

In some embodiments, one or more inertial measurement signals output by inertial measurement unit 121 may be supplied to communication interface 128 and thence to acoustic imaging instrument 110 where any desired processing may occur. In other embodiments, the one or more inertial measurement signals output by inertial measurement unit 121 may be supplied to signal processor 126 (instead of directly to communications interface 128) which may process the inertial measurement signal(s) as desired and provide processed inertial measurement signal(s) to communication interface 128, and thence to acoustic imaging instrument 110.

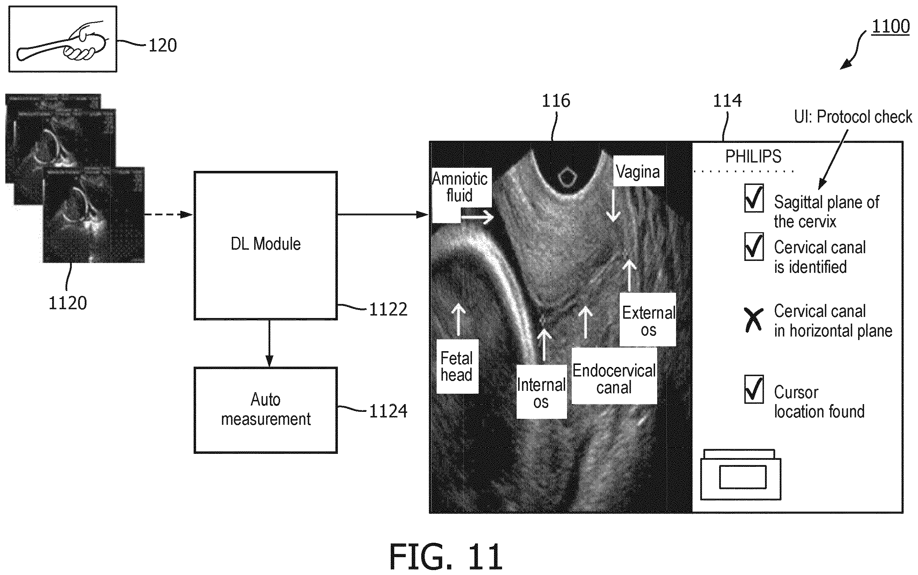

FIG. 11 illustrates an example operation of an acoustic imaging apparatus such as acoustic imaging instrument 110 during a scan session for measuring cervical length.

FIG. 11 shows a deep learning module 1122, implemented as a portion of a software program, executed by processor 112 in a scan session for measuring cervical length. Deep learning module 1122 is associated with an auto measurement software program 1124 which may be executed by processor 112 to acquire acoustic images of a cervix for measuring cervical length in response to one or more criteria or system configuration settings for automatic CL measurement being activated. These settings may include (but are not limited to), a tissue specific preset (TSP) setting, a user-specific profile that indicates the intention to perform a cervix measurement, the activation of a transvaginal sonography (TVS) probe, etc..

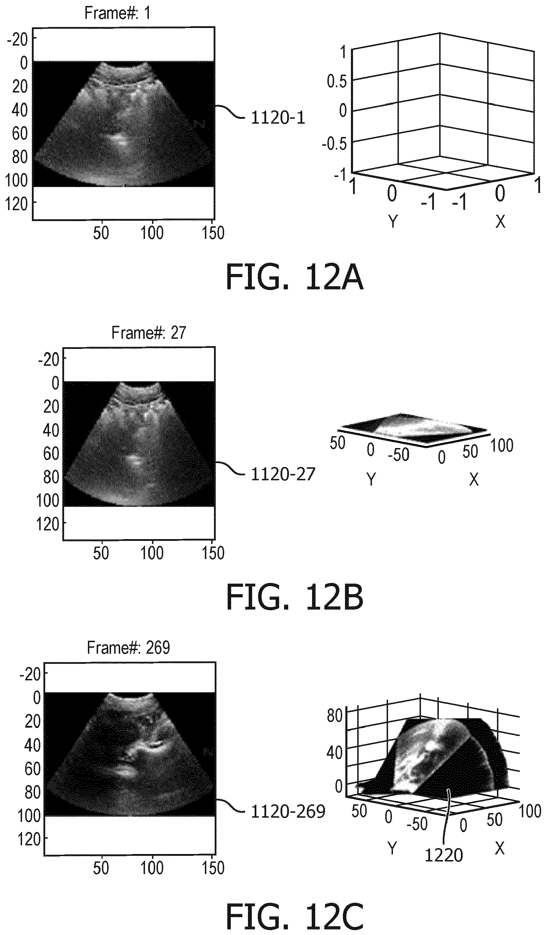

During a scan session, acoustic imaging instrument 110 may receive one or more image signals from acoustic probe 120, may process the image signal(s) to produce acoustic image data as acoustic probe 120 scans different views of area of interest 10 in different 2D planes, and may construct a three dimensional (3D) volume 1220 of area of interest 10 from the acoustic image data and the received inertial measurement signal, as shown in FIGs. 12A, 12B and 12C.

FIGs. 12A, 12B and 12B illustrate an example operation of a process of constructing a 3D volume 1220 from a series of acoustic images 1220, The process starts with a first two dimensional image or frame 1120-1 taken at a first image plane, shown on the left hand side of FIG. 12A, proceeding through a 27th image or frame 1120-27 taken at a 27th image plane, shown on the left hand side of FIG. 12B and then proceeding to a 269th image or frame 1120-269 taken at a 269th image plane, shown on the left hand side of FIG. 12C. Of course a plurality of other acoustic images or frames are taken, but not shown in FIGs. 12A, 12B and 12C for simplifying the illustration.

Acoustic imaging instrument 110 may then qualify one or more of the acoustic images 1120 and corresponding plane within the 3D volume 1220 for making a candidate cervical length measurement. In particular, deep learning module 1122 may employ a standard deep learning network architecture such as a classic convolutional neural network (CNN), a You Only Look Once (YOLO) neural network, or a U-Net Convolutional network (U-net) to perform tasks such as classification, regression, object detection and segmentation for acoustic images formed by acoustic imaging instrument 110 from image signals of the cervix received from acoustic probe 120.

Deep learning module may also be implemented as a hardware circuit rather than software executed by processor 112.

In particular, to guide the user to the optimal imaging plane based on anatomical landmarks, deep learning module 1122 may employ one or more qualifying anatomical landmarks which qualify image planes of the three dimensional volume, and/or one or more disqualifying anatomical landmarks which disqualify image planes of the three dimensional volume of area of interest 10. Usually, certain (qualifying) anatomical landmarks are required to achieve an optimal view, while the presence of other (disqualifying) anatomical landmarks automatically disqualify the view as sub-optimal. For example, deep learning module 1122 may implement a YOLO network which enables object recognition in images, and may employ the YOLO network to search for the presence of the qualifying and disqualifying anatomical landmarks in image planes of the 3D volume.

Deep learning module 1122 may be trained with the following inputs for measurement guidance: (1) a series of B-mode acoustic images; (2) labels for optimal and suboptimal views; and (3) labelled anatomical regions/ landmarks.

In a clinical operation scenario, a sonographer may employ acoustic probe 120 and acoustic imaging instrument 110 during a scan session for measuring cervical length as follows. The sonographer places the acoustic probe 120 in suitable position so as to view the cervix. The acquired B-mode images are applied to deep learning module 1122 in real time. Then deep learning module 1122 can determine, among other things: (1) whether a qualified view is identified based on whether the presence or absence of the qualifying and qualifying anatomical landmarks in an image; (2) whether right amount pressure is applied or not; and (3) the correct caliper location for making a cervical length measurement; etc.

Output of deep learning module 1122 may be presented as an overlay on user interface 114. FIG. 11 shows some check boxes in user interface 114 which may be checked off as each of these items are determined to provide feedback to the sonographer.

During a scan session as described above, all the qualified or best views identified are marked for candidate cervical length measurement, a candidate cervical length measurement is automatically performed, and the shortest candidate cervical length measurement among all the qualified or best views is selected as the measured cervical length for the scan session. For pressure measurements, deep learning module 1122 can use the shape of the cervix as an anatomical landmark.

FIG. 13 illustrates major operations in an example embodiment of an algorithm 1300 for determining the cervical length of a cervix.

An operation 1310 includes performing a real-time ("live") 2D acoustic imaging scan of area of interest 10, including a cervix.

An operation 1320 includes activating an automatic cervical length measurement mode during the live acoustic imaging scan session, based on the system configuration settings automatic CL measurement mode will be activated. These settings can include (but not limited to) the tissue specific preset (TSP) setting, a user-specific profile that indicates the intention to perform a cervix measurement, the activation of a transvaginal transducer etc.

Operation 1330 includes constructing a 3D volume from the series of 2D images captured in operation 1310 as the user or sonographer maneuvers acoustic probe 120. Optionally, in some embodiments, the user can be tasked with performing specific probe maneuvers (e.g., rotational maneuvers) to ensure that additional 3D segments are captured. The pose information for each acoustic image may be obtained in an operation 1335 from an inertial measurement signal produced by IMU 121. IMU 121 provides pose measurements relative to a previous measurement or 2D acoustic image. In other words, during a transient motion of the acoustic probe 120, the signal output by IMU 121 can be used to construct a 3D volume from the individual 2D image frames.

Operation 1340 includes identifying an image plane for measurement of cervical length. In particular, from the 3D volume constructed in operation 1330, an appropriate image plane is identified from the volume. To identify an appropriate image plane, in operation 1345 each plane may be passed through the deep learning module as described above with respect to FIG. 11, to identify an image plane which meets all the criteria for a correct measurement of cervical length. With this approach multiple image planes which are qualified for CL measurement may be identified. In some embodiments, an optimal image plane may be determined, for example by weighting a plurality of qualifying landmarks and disqualifying landmarks, and finding the image plane which most closely matches the qualifying landmarks and least closely matches the disqualifying landmarks. In some embodiments, the optimal image plane may be an oblique plane within the 3D volume.

Operation 1350 includes making an automatic measurement of cervical length. That is, once image planes are identified in operation 1340 for measurement of cervical length, correct caliper points for measuring a candidate cervical length in the image plane are identified. In some embodiments, an operation 1355 may include processor 112 performing image segmentation and object detection for the qualified image plane to obtain the candidate cervical length. In some embodiments, processor 112 is configured to perform image segmentation by applying the image data for the qualified image plane to a You Only Look Once (YOLO) neural network. In some embodiments, processor 112 is configured to perform object detection for the qualified image plane by applying the image data for the qualified image plane to a U-Net Convolutional network. However, in other embodiments, other techniques may be employed.

An operation 1360 includes displays a temporal graph or trace: In standard clinical practice, three or more candidate cervical lengths are obtained for the clinical diagnosis, in a given scan session. The clinical goal is to capture the shortest candidate cervical length in a given scan session, out of all the measurements made in that session, as the measured cervical length for that scan session. The rationale for making multiple measurements is, as stated above, that the estimated cervix length can change due to a number of reasons, including patient motion, breathing, probe motion etc. Based on these clinical criteria, in operation 1360 acoustic imaging system 100 executing algorithm 1300 displays a trace of candidate cervical length measurements over time on qualified frames and marks the best shortest cervical length on a graph displayed on display device 116 via user interface 114.

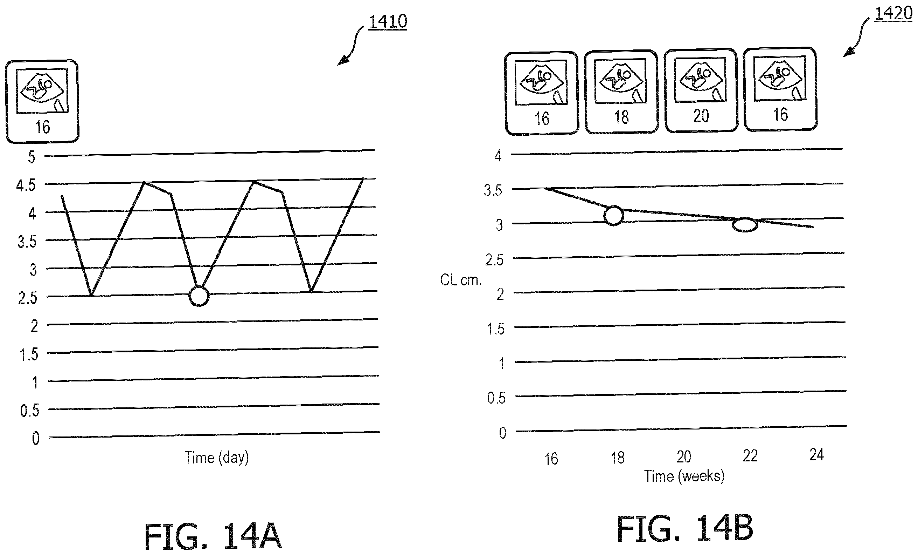

FIG. 14A illustrates an example of a graph 1410 which may be displayed on display device 116 via user interface 114 to show candidate cervical lengths and to indicate the measured cervical length for a scan session. Beneficially, the acoustic image corresponding to the cervical length measurement also may be displayed on display device 116 to provide context to the sonographer or user. In an operation 1365, the results, including the measured cervical length, may be archived in a nonvolatile storage device or memory of an electronic medical record (EMR) system for generating a longitudinal result which acoustic imaging system 100 may present to the sonographer or user in a follow up scan session.

During a follow up scan session, operations 1310 -1365 may be performed again to obtain a new cervical length measurement. The acoustic images stored from earlier scan sessions can be retrieved, and (optionally) image matching can be performed with the acoustic images from the current live session. This ensures similar image planes are used for the various cervical length measurements over time to yield consistent results.

FIG. 14B illustrates an example of a graph 1420 which may be displayed on display device 116 via user interface 114 to show a progression of measured cervical lengths over time from multiple scan sessions during a pregnancy. This feature allows the clinician to observe the trend in cervical length changes for a patient, along with corresponding acoustic images. When a user clicks on a particular week number in graph 1420, the trace of the cervical length measurement for that particular scan session may be displayed, similar to the example graph 1410 depicted in FIG. 14A.

It should be understood that the order of various operations in FIG. 13 may be changed or rearranged, and indeed some operations may actually be performed in parallel with one or more other operations. In that sense, FIG. 13 may be better viewed as a numbered list of operations rather than an ordered sequence.

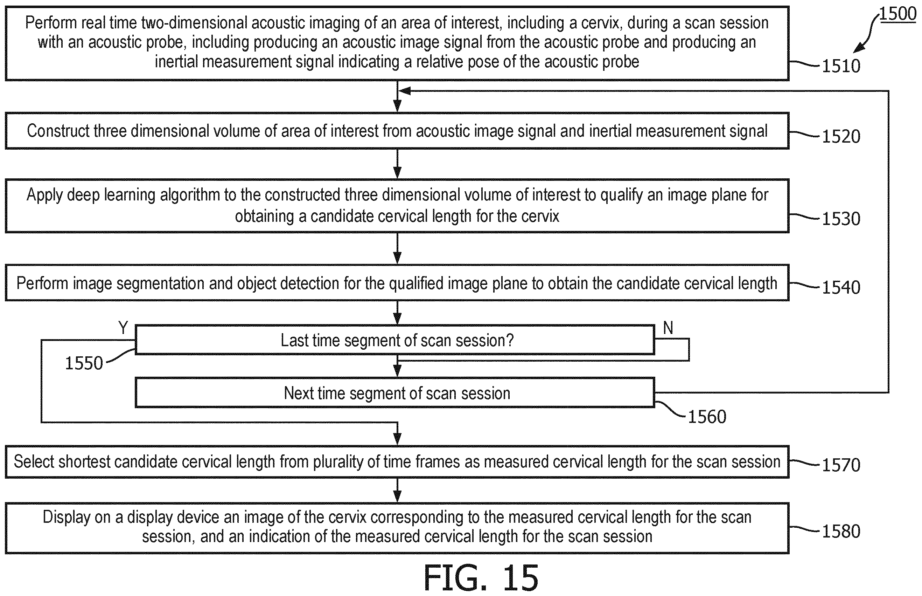

FIG. 15 illustrates a flowchart of an example embodiment of a method 1500 of determining the cervical length of a cervix which may be performed using the acoustic imaging system 100 as described above.

An operation 1510 may include performing real time two-dimensional acoustic imaging of an area of interest, including a cervix, during a scan session with an acoustic probe, including producing an acoustic image signal from the acoustic probe and producing an inertial measurement signal indicating a pose of the acoustic probe.

An operation 1520 may include constructing a three dimensional volume of the area of interest from the acoustic image signal and the inertial measurement signal.

An operation 1530 may include applying a deep learning algorithm to the constructed three dimensional volume of interest to qualify an image plane for obtaining a candidate cervical length for the cervix.

An operation 1540 may include performing image segmentation and object detection for the qualified image plane to obtain the candidate cervical length, and the candidate cervical length is stored in memory.

An operation 1550 may include determining whether the last time segment of a scan session has been processed. In some embodiments, a threshold number (e.g., three) of candidate cervical length measurements may be established, and the last time segment may be determined as a time segment when the threshold has been reached. In other embodiments, the last time segment may be determined as when the sonographer removes the acoustic probe from the area if interest, or presses a button, or otherwise indicates that the scan session is complete.

If it is determined in operation 1550 that the last time segment has not yet been processed, then the method proceeds to an operation 1560 wherein the next time segment is scan session is collected. Then the method returns to operation 1520 and continues to process additional acoustic images to determine additional candidate cervical lengths in subsequent time segments. If it is determined in operation 1550 that the last time segment has been processed, then the method proceeds to operation 1570.

Operation 1570 occurs when all of the candidate cervical lengths for a scan session have been obtained, and may include selecting the shortest candidate cervical length from the plurality of time frames as the measured cervical length for the scan session.

An operation 1580 may include displaying on a display device an image of the cervix in the qualified plane produced from the acoustic image signal, together with an indication of the measured cervical length for the scan session.

It should be understood that the order of various operations in FIG. 15 may be changed or rearranged, and indeed some operations may actually be performed in parallel with one or more other operations. In that sense, FIG. 15 may be better viewed as a numbered list of operations rather than an ordered sequence.

While preferred embodiments are disclosed in detail herein, many variations are possible which remain within the concept and scope of the invention. Such variations would become clear to one of ordinary skill in the art after inspection of the specification, drawings and claims herein. The invention therefore is not to be restricted except within the scope of the appended claims.

## Claims

1. A system (100), comprising:
an acoustic probe (120), the acoustic probe having an array (122) of acoustic transducer elements;
an inertial measurement circuit (121), the inertial measurement circuit configured to provide an inertial measurement signal indicating a pose of the acoustic probe; and
an acoustic imaging instrument (110) connected to the acoustic probe and configured to provide transmit signals to least some of the acoustic transducer elements to cause the array of acoustic transducer elements to transmit an acoustic probe signal (15) to an area of interest (10) including a cervix, and further configured to produce acoustic images of the area of interest in response to acoustic echoes received by the acoustic probe from the area of interest in response to the acoustic probe signal, the acoustic imaging instrument including:
a display device (116);
a communication interface (115) configured to receive one or more image signals from the acoustic probe produced from the acoustic echoes from the area of interest, and to receive the inertial measurement signal; and
a processor (112, 900), and associated memory (111), configured to:
for each of a plurality of time frames (1120-1/1120/27/1120-269) in a scan session:
construct (1520) a three dimensional volume (1220) of the area of interest from the one or more image signals and the received inertial measurement signal,
apply (1530) a deep learning algorithm (1122) to the constructed three dimensional volume of the area of interest to qualify (1340) an image plane for obtaining a candidate cervical length for the cervix, and
perform (1540) image segmentation and object detection for the qualified image plane to obtain (1350) the candidate cervical length, and
select (1570) a shortest candidate cervical length from the plurality of time frames as a measured cervical length for the scan session,
wherein the processor is configured to control the display device to display an image of the cervix corresponding to the measured cervical length for the scan session, and an indication of the measured cervical length for the scan session.

2. The system (100) of claim 1, wherein the processor is configured to control the display device to display a graph (1410) showing the candidate cervical lengths and to display the indication of the measured cervical length for the scan session on the graph.

3. The system (100) of claim 1, wherein the processor is configured to store in a nonvolatile memory device (932, 934, 935, 936) the measured cervical length for the scan session and a date of the scan session.

4. The system (100) of claim 3,
wherein the nonvolatile memory device is configured to store a plurality of measured cervical lengths for a plurality of scan sessions performed at corresponding times, and
wherein the processor is configured to cause the display to display a graph plotting the cervical lengths for the scan sessions against the corresponding times.

5. The system (100) of claim 1, wherein the processor is configured to generate image data for the qualified image plane and to perform image segmentation by applying the image data for the qualified image plane to a You Only Look Once (YOLO) neural network.

6. The system (100) of claim 1, wherein the processor is configured to generate image data for the qualified image plane and to perform object detection for the qualified image plane by applying the image data for the qualified image plane to a U-Net Convolutional network.

7. The system (100) of claim 1,
wherein the processor is configured to generate image data for a plurality of image planes of the three dimensional volume, and
wherein the deep learning algorithm employs one or more qualifying anatomical landmarks which qualify image planes of the three dimensional volume, and employs one or more disqualifying anatomical landmarks which disqualify image planes of the three dimensional volume.

8. The system (100) of claim 7, wherein a first cervical shape is employed as one of the disqualifying anatomical landmarks and a second cervical shape is employed as one of the qualifying anatomical landmarks.

9. The system (100) of claim 1, wherein the processor is configured to generate image data for a plurality of image planes of the three dimensional volume, and wherein the deep learning algorithm applies the image data to one of a convolutional neural network (CNN), a You Only Look Once (YOLO) neural network, or a U-Net Convolutional network.

10. A method (1500), comprising:
performing (1510) real time two-dimensional acoustic imaging of an area of interest (10) during a scan session, including a cervix, with an acoustic probe (120), including producing one or more image signals of the area of interest and producing an inertial measurement signal indicating a pose of the acoustic probe;
for each of a plurality of time frames in the scan session:
constructing (1520) a three dimensional volume (1220) of the area of interest from the one or more image signals and the inertial measurement signal,
applying a (1530) deep learning algorithm to the constructed three dimensional volume of the area of interest to qualify an image plane for obtaining a candidate cervical length for the cervix, and
performing (1540) image segmentation and object detection for the qualified image plane to obtain the candidate cervical length; and
selecting (1570) a shortest candidate cervical length from the plurality of time frames as a measured cervical length for the scan session; and
displaying (1570) on a display device (116) an image of the cervix corresponding to the measured cervical length for the scan session, and an indication of the measured cervical length for the scan session.

11. The method (1500) of claim 10, further comprising displaying a graph (1410) showing the candidate cervical lengths and displaying the indication of the measured cervical length for the scan session on the graph.

12. The method (1500) of claim 10, further comprising storing the measured cervical length for the scan session and a date of the scan session in a nonvolatile memory device (932, 934, 935, 936).

13. The method (1500) of claim 12, further comprising:
storing in the nonvolatile memory device (932, 934, 935, 936) a plurality of measured cervical lengths for a plurality of scan sessions performed at corresponding times; and
displaying on the display device a graph (1410) plotting the cervical lengths for the scan sessions against the corresponding times.

14. The method (1500) of claim 10, further comprising generating image data for the qualified image plane and performing image segmentation by applying the image data for the qualified image plane to a You Only Look Once (YOLO) neural network.

15. The method (1500) of claim 10, further comprising:
generating image data for the qualified image plane; and
performing object detection for the qualified image plane by applying the image data for the qualified image plane to a U-Net Convolutional network.

## Patentansprüche

1. System (100), umfassend:
eine akustische Sonde (120), wobei die akustische Sonde eine Anordnung (122) von akustischen Wandlerelementen aufweist;
eine Trägheitsmessschaltung (121), wobei die Trägheitsmessschaltung konfiguriert ist, um ein Trägheitsmesssignal bereitzustellen, das eine Lage der akustischen Sonde angibt; und
ein akustisches Bildgebungsinstrument (110), das mit der akustischen Sonde verbunden und konfiguriert ist, um Übertragungssignale an mindestens einige der akustischen Wandlerelemente bereitzustellen, um zu bewirken, dass die Anordnung von akustischen Wandlerelementen ein akustisches Sondensignal (15) an einen Bereich von Interesse (10) einschließlich eines Gebärmutterhalses überträgt, und das ferner konfiguriert ist, um akustische Bilder des Bereichs von Interesse als Reaktion auf akustische Echos zu erzeugen, die von der akustischen Sonde als Reaktion auf das akustische Sondensignal von dem Bereich von Interesse empfangen werden, wobei das akustische Bildgebungsinstrument Folgendes beinhaltet:
eine Anzeigevorrichtung (116);
eine Kommunikationsschnittstelle (115), die konfiguriert ist, um ein oder mehrere Bildsignale von der akustischen Sonde zu empfangen, die von den akustischen Echos aus dem Bereich von Interesse erzeugt werden, und um das Trägheitsmesssignal zu empfangen; und
einen Prozessor (112, 900) und assoziierten Speicher (111), der zu Folgendem konfiguriert ist:
für jeden einer Vielzahl von Zeitrahmen (1120-1/1120/27/1120-269) in einer Scan-Sitzung:
Konstruieren (1520) eines dreidimensionalen Volumens (1220) des Bereichs von Interesse aus dem einen oder den mehreren Bildsignalen und dem empfangenen Trägheitsmesssignal,
Anwenden (1530) eines Deep-Learning-Algorithmus (1122) auf das konstruierte dreidimensionale Volumen des Bereichs von Interesse, um eine Bildebene zu qualifizieren (1340), um eine Kandidaten-Gebärmutterhalslänge zu erlangen, und
Ausführen (1540) einer Bildsegmentierung und Objekterkennung für die qualifizierte Bildebene, um die Kandidaten-Gebärmutterhalslänge zu erlangen (1350), und
Auswählen (1570) einer kürzesten Kandidaten-Gebärmutterhalslänge aus der Vielzahl von Zeitrahmen als gemessene Gebärmutterhalslänge für die Scan-Sitzung,
wobei der Prozessor konfiguriert ist, um die Anzeigevorrichtung zu steuern, um ein Bild des Gebärmutterhalses entsprechend der gemessenen Gebärmutterhalslänge für die Scan-Sitzung und eine Angabe der gemessenen Gebärmutterhalslänge für die Scan-Sitzung anzuzeigen.

2. System (100) nach Anspruch 1, wobei der Prozessor konfiguriert ist, um die Anzeigevorrichtung zu steuern, um ein Diagramm (1410) anzuzeigen, das die Kandidaten-Gebärmutterhalslänge zeigt, und um die Angabe der gemessenen Gebärmutterhalslänge für die Scan-Sitzung auf dem Diagramm anzuzeigen.

3. System (100) nach Anspruch 1, wobei der Prozessor konfiguriert ist, um die gemessene Gebärmutterhalslänge für die Scan-Sitzung und ein Datum der Scan-Sitzung in einer nichtflüchtigen Speichervorrichtung (932, 934, 935, 936) zu speichern.

4. System (100) nach Anspruch 3, wobei die nichtflüchtige Speichervorrichtung konfiguriert ist, um eine Vielzahl von gemessenen Gebärmutterhalslängen für eine Vielzahl von zu entsprechenden Zeitpunkten ausgeführten Abtastsitzungen zu speichern, und
wobei der Prozessor konfiguriert ist, um die Anzeige zu veranlassen, ein Diagramm anzuzeigen, in dem die Gebärmutterhalslängen für die Scan-Sitzungen gegen die entsprechenden Zeitpunkte aufgetragen sind.

5. System (100) nach Anspruch 1, wobei der Prozessor konfiguriert ist, um Bilddaten für die qualifizierte Bildebene zu erzeugen und eine Bildsegmentierung auszuführen, indem er die Bilddaten für die qualifizierte Bildebene auf ein neuronales Netzwerk You Only Look Once (YOLO) anwendet.

6. System (100) nach Anspruch 1, wobei der Prozessor konfiguriert ist, um Bilddaten für die qualifizierte Bildebene zu erzeugen und eine Objekterkennung für die qualifizierte Bildebene auszuführen, indem er die Bilddaten für die qualifizierte Bildebene auf ein faltendes neuronales U-Net-Netzwerk anwendet.

7. System (100) nach Anspruch 1, wobei der Prozessor konfiguriert ist, um Bilddaten für eine Vielzahl von Bildebenen des dreidimensionalen Volumens zu erzeugen, und
wobei der Deep-Learning-Algorithmus eine oder mehrere qualifizierende anatomische Leitstrukturen einsetzt, die die Bildebenen des dreidimensionalen Volumens qualifizieren, und eine oder mehrere disqualifizierende anatomische Leitstrukturen einsetzt, die die Bildebenen des dreidimensionalen Volumens disqualifizieren.

8. System (100) nach Anspruch 7, wobei eine erste Gebärmutterhalsform als eine der disqualifizierenden anatomischen Leitstrukturen eingesetzt wird und eine zweite Gebärmutterhalsform als eine der qualifizierenden anatomischen Leitstrukturen eingesetzt wird.

9. System (100) nach Anspruch 1, wobei der Prozessor konfiguriert ist, um Bilddaten für eine Vielzahl von Bildebenen des dreidimensionalen Volumens zu erzeugen, und wobei der Deep-Learning-Algorithmus die Bilddaten auf eines von einem faltenden neuronalen Netzwerk (CNN), einem neuronalen Netzwerk You Only Look Once (YOLO) oder einem faltenden neuronalen U-Net-Netzwerk anwendet.

10. Verfahren (1500), umfassend:
Ausführen (1510) einer zweidimensionalen akustischen Echtzeit-Bildgebung eines Bereichs von Interesse (10) während einer Scan-Sitzung, einschließlich eines Gebärmutterhalses, mit einer akustischen Sonde (120), einschließlich Erzeugen eines oder mehrerer Bildsignale des Bereichs von Interesse und Erzeugen eines Trägheitsmesssignals, das die Lage der akustischen Sonde angibt;
für jeden einer Vielzahl von Zeitrahmen in der Scan-Sitzung:
Konstruieren (1520) eines dreidimensionalen Volumens (1220) des Bereichs von Interesse aus dem einen oder den mehreren Bildsignalen und dem Trägheitsmesssignal, Anwenden (1530) eines Deep-Learning-Algorithmus auf das konstruierte dreidimensionale Volumen des Bereichs von Interesse, um eine Bildebene zu qualifizieren, um eine Kandidaten-Gebärmutterhalslänge zu erlangen; und
Ausführen (1540) einer Bildsegmentierung und Objekterkennung für die qualifizierte Bildebene, um die Kandidaten-Gebärmutterhalslänge zu erlangen; und
Auswählen (1570) einer kürzesten Kandidaten-Gebärmutterhalslänge aus der Vielzahl von Zeitrahmen als gemessene Gebärmutterhalslänge für die Scan-Sitzung; und
Anzeigen (1570), auf einer Anzeigevorrichtung (116), eines Bilds des Gebärmutterhalses entsprechend der gemessenen Gebärmutterhalslänge für die Scan-Sitzung, und einer Angabe der gemessenen Gebärmutterhalslänge für die Scan-Sitzung.

11. Verfahren (1500) nach Anspruch 10, ferner umfassend ein Anzeigen eines Diagramms (1410), das die Kandidaten-Gebärmutterhalslängen zeigt, und Anzeigen der Angabe der gemessenen Gebärmutterhalslänge für die Scan-Sitzung in dem Diagramm.

12. Verfahren (1500) nach Anspruch 10, ferner umfassend das Speichern der gemessenen Gebärmutterhalslänge für die Scan-Sitzung und eines Datums der Scan-Sitzung in einer nichtflüchtigen Speichervorrichtung (932, 934, 935, 936).

13. Verfahren (1500) nach Anspruch 12, ferner umfassend:
Speichern, in der nichtflüchtigen Speichervorrichtung (932, 934, 935, 936), einer Vielzahl von gemessenen Gebärmutterhalslängen für eine Vielzahl von Scan-Sitzungen, die zu entsprechenden Zeitpunkten ausgeführt werden; und
Anzeigen, auf der Anzeigevorrichtung, eines Diagramms (1410), in dem die Gebärmutterhalslängen für die Scan-Sitzungen gegen die entsprechenden Zeitpunkte aufgetragen sind.

14. Verfahren (1500) nach Anspruch 10, ferner umfassend ein Erzeugen von Bilddaten für die qualifizierte Bildebene und ein Ausführen einer Bildsegmentierung durch Anwenden der Bilddaten für die qualifizierte Bildebene auf ein neuronales Netzwerk You Only Look Once (YOLO).

15. Verfahren (1500) nach Anspruch 10, ferner umfassend:
Erzeugen von Bilddaten für die qualifizierte Bildebene; und
Ausführen einer Objekterkennung für die qualifizierte Bildebene durch Anwenden der Bilddaten für die qualifizierte Bildebene auf ein faltendes neuronales U-Net-Netzwerk.

## Revendications

1. Système (100) comprenant:
une sonde acoustique (120), la sonde acoustique ayant un réseau (122) d'éléments transducteurs acoustiques;
un circuit de mesure inertielle (121), le circuit de mesure inertielle étant configuré pour fournir un signal de mesure inertielle indiquant la position de la sonde acoustique; et
un outil d'imagerie acoustique (110) connecté à la sonde acoustique et configuré pour fournir des signaux de transmission à au moins certains des éléments transducteurs acoustiques pour amener le réseau d'éléments transducteurs acoustiques à transmettre un signal de sonde acoustique (15) à une zone d'intérêt (10) comprenant un col de l'utérus, et configuré en outre pour produire des images acoustiques de la zone d'intérêt en réponse aux échos acoustiques reçus par la sonde acoustique de la zone d'intérêt en réponse au signal de sonde acoustique, l'outil d'imagerie acoustique comprenant:
un dispositif d'affichage (116);
une interface de communication (115) configurée pour recevoir un ou plusieurs signaux d'image de la sonde acoustique produits à partir des échos acoustiques de la zone d'intérêt, et pour recevoir le signal de mesure inertielle; et
un processeur (112, 900) et une mémoire associée (111), configurés pour:
pour chacune de plusieurs images temporelles (1120-1/1120/27/1120-269) dans une session de balayage:
construire (1520) un volume tridimensionnel (1220) de la zone d'intérêt à partir d'un ou de plusieurs signaux d'image et du signal de mesure inertielle reçu, appliquer (1530) un algorithme d'apprentissage profond (1122) au volume tridimensionnel construit de la zone d'intérêt pour qualifier (1340) un plan d'image afin d'obtenir une longueur cervicale candidate pour le col de l'utérus, et effectuer (1540) la segmentation de l'image et la détection d'objets pour le plan d'image qualifié afin d'obtenir (1350) la longueur cervicale candidate, et sélectionner (1570) une longueur cervicale candidate la plus courte parmi la pluralité d'images temporelles en tant que longueur cervicale mesurée pour la session de balayage, dans lequel le processeur est configuré pour commander le dispositif d'affichage afin d'afficher une image du col de l'utérus correspondant à la longueur cervicale mesurée pour la session de balayage, et une indication de la longueur cervicale mesurée pour la session de balayage.

2. Système (100) selon la revendication 1, dans lequel le processeur est configuré pour commander le dispositif d'affichage afin d'afficher un graphique (1410) montrant les longueurs cervicales candidates et d'afficher l'indication de la longueur cervicale mesurée pour la session de balayage sur le graphique.

3. Système (100) selon la revendication 1, dans lequel le processeur est configuré pour stocker dans un dispositif de mémoire non volatile (932, 934, 935, 936) la longueur cervicale mesurée pour la session de balayage et la date de la session de balayage.

4. Système (100) selon la revendication 3,
dans lequel le dispositif de mémoire non volatile est configuré pour stocker une pluralité de longueurs cervicales mesurées pour une pluralité de sessions de balayage effectuées à des moments correspondants, et dans lequel le processeur est configuré pour que l'écran affiche un graphique représentant les longueurs cervicales pour les sessions de balayage en fonction des moments correspondants.

5. Système (100) selon la revendication 1, dans lequel le processeur est configuré pour générer des données d'image pour le plan d'image qualifié et pour effectuer une segmentation d'image en appliquant les données d'image pour le plan d'image qualifié à un réseau neuronal You Only Look Once (YOLO).

6. Système (100) selon la revendication 1, dans lequel le processeur est configuré pour générer des données d'image pour le plan d'image qualifié et pour effectuer une détection d'objet pour le plan d'image qualifié en appliquant les données d'image pour le plan d'image qualifié à un réseau convolutionnel U-Net.

7. Système (100) selon la revendication 1,
dans lequel le processeur est configuré pour générer des données d'image pour une pluralité de plans d'image du volume tridimensionnel, et dans lequel l'algorithme d'apprentissage profond utilise un ou plusieurs repères anatomiques qualifiants qui qualifient les plans d'image du volume tridimensionnel, et utilise un ou plusieurs repères anatomiques disqualifiants qui disqualifient les plans d'image du volume tridimensionnel.

8. Système (100) selon la revendication 7, dans lequel une première forme cervicale est utilisée comme l'un des repères anatomiques disqualifiants et une deuxième forme cervicale est utilisée comme l'un des repères anatomiques qualifiants.

9. Système (100) selon la revendication 1, dans lequel le processeur est configuré pour générer des données d'image pour une pluralité de plans d'image du volume tridimensionnel, et dans lequel l'algorithme d'apprentissage profond applique les données d'image à l'un des réseaux neuronaux convolutionnels (CNN), un réseau neuronal You Only Look Once (YOLO), ou un réseau convolutionnel U-Net.

10. Méthode (1500) comprenant:
la réalisation (1510) d'une imagerie acoustique bidimensionnelle en temps réel d'une zone d'intérêt (10) au cours d'une session de balayage, y compris un col de l'utérus, à l'aide d'une sonde acoustique (120), y compris la production d'un ou plusieurs signaux d'image de la zone d'intérêt et la production d'un signal de mesure inertielle indiquant la position de la sonde acoustique;
pour chacun des images temporelles de la session de balayage:
construire (1520) un volume tridimensionnel (1220) de la zone d'intérêt à partir d'un ou plusieurs signaux d'image et du signal de mesure inertielle,
appliquer un algorithme (1530) d'apprentissage profond au volume tridimensionnel construit de la zone d'intérêt afin de qualifier un plan d'image pour obtenir une longueur cervicale candidate pour le col de l'utérus, et
effectuer (1540) une segmentation d'image et une détection d'objet pour le plan d'image qualifié afin d'obtenir la longueur cervicale candidate; et
sélectionner (1570) une longueur cervicale candidate la plus courte parmi la pluralité d'images temporelles en tant que longueur cervicale mesurée pour la session de balayage; et
afficher (1570) sur un dispositif d'affichage (116) une image du col de l'utérus correspondant à la longueur cervicale mesurée pour la session de balayage, et une indication de la longueur cervicale mesurée pour la session de balayage.

11. Méthode (1500) selon la revendication 10, comprenant en outre l'affichage d'un graphique (1410) montrant les longueurs cervicales candidates et l'affichage de l'indication de la longueur cervicale mesurée pour la session de balayage sur le graphique.

12. Méthode (1500) selon la revendication 10, comprenant en outre le stockage de la longueur cervicale mesurée pour la session de balayage et une date de la session de balayage dans un dispositif de mémoire non volatile (932, 934, 935, 936).

13. Méthode (1500) selon la revendication 12, comprenant en outre:
le stockage dans le dispositif de mémoire non volatile (932, 934, 935, 936) d'une pluralité de longueurs cervicales mesurées pour une pluralité de sessions de balayage effectuées à des moments correspondants; et
l'affichage sur le dispositif d'affichage d'un graphique (1410) représentant les longueurs cervicales pour les sessions de balayage en fonction des moments correspondantes.

14. Méthode (1500) selon la revendication 10, comprenant en outre la génération de données d'image pour le plan d'image qualifié et la réalisation d'une segmentation d'image en appliquant les données d'image pour le plan d'image qualifié à un réseau neuronal You Only Look Once (YOLO).

15. Méthode (1500) selon la revendication 10, comprenant en outre:
la génération des données d'image pour le plan d'image qualifié; et
la détection d'objets pour le plan d'image qualifié en appliquant les données d'image pour le plan d'image qualifié à un réseau convolutionnel U-Net.
